(19) Europäisches Patentamt
European Patent Office
Office européen des brevets

(11) Publication number: 0 251 489 B1

(12) # EUROPEAN PATENT SPECIFICATION

(45) Date of publication of patent specification: 22.07.92

(51) Int. Cl.5: C12P 19/32, C12P 39/00, //C12N15/52

(21) Application number: 87304689.0

(22) Date of filing: 27.05.87

(54) **Process for producing 5'-guanylic acid.**

(30) Priority: 02.06.86 JP 127720/86

(43) Date of publication of application:
07.01.88 Bulletin 88/01

(45) Publication of the grant of the patent:
22.07.92 Bulletin 92/30

(84) Designated Contracting States:
DE FR GB

(56) References cited:
EP-A- 0 185 092

CHEMICAL ABSTRACTS, vol. 76, no. 7, 14th February 1972, page 218, no. 32874y, Columbus, Ohio, US; & JP-A-71 39 070 (KYOWA FERMENTATION INDUSTRY CO., LTD) 17-11-1971

MICROBIOLOGY ABSTRACTS. B. BACTERIOLOGY, vol. 6, no. 3, 13th October 1971, page 1, no. B10747; A. FURUYA et al.: "Conversion of 5'xanthylic acid to guanine and guanine nucleotides by a mutant of Brevibacterium ammoniagenes", & BIOTECHNOL. AND BIOENG., 13, 229-240 (1971)

(73) Proprietor: KYOWA HAKKO KOGYO CO., LTD.
6-1, Ohte-Machi Itchome
Chiyoda-ku Tokyo(JP)

(72) Inventor: Fujio, Tatsuro
6-29-1, Sagamidai
Sagamihara-shi Kanagawa-ken(JP)
Inventor: Maruyama, Akihiko
Hashimoto-Heights 1-2 3150, Noborito Tama-ku
Kawasaki-shi Kanagawa-ken(JP)

(74) Representative: Lambert, Hugh Richmond et al
D. YOUNG & CO. 10 Staple Inn
London, WC1V 7RD(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

AGRIC. BIOL. CHEM., vol. 43, no. 8, 1979, pages 1739-1744; H. MATSUI et al.: "Production of guanosine by psicofuranine and decoyinine resistant mutants of Bacillus subtilis"

CHEMICAL ABSTRACTS, vol. 101, no. 4, August 1984, page 479, no. 53221h, Columbus, Ohio, US; T. FUJIO et al.: "Production of nucleic acid-related substances by fermentative processes. Part 52. Production of 5'-guanylic acid by enzymatic conversion of 5'-xanthylic acid", & J. FERMENT. TECHNOL. 1984, 62(2), 131-7

AGRIC. BIOL. CHEM., vol. 50, no. 7, July 1986, pages 1879-1884; A. MARUYAMA et al.: "Construction of a plasmid for high level expression of XMP aminase in Escherichia coli"

## Description

The present invention relates to a process for producing 5'-guanylic acid (hereinafter referred to as "GMP").

GMP is in great demand as a seasoning, and the development of a process for producing GMP at lower cost has been desired in the field of food industry.

So far, known processes for producing GMP include (1) a process of enzymatically decomposing ribonucleic acid extracted from yeast cells, (2) a process of chemically phosphorylating guanosine produced by fermentation, (3) a process of converting 5'-xanthylic acid (hereinafter referred to as "XMP") produced by fermentation into GMP by means of bacteria belonging to the genus Brevibacterium or Corynebacterium (Japanese Published Examined Patent Application No. 39069/71 corresponding to US-A-3,592,733, and Japanese Published Unexamined Patent Application No. 78697/84), and (4) a process of converting XMP into GMP using an Escherichia coli transformant transformed with a recombinant DNA vector comprising a DNA fragment containing a gene coding for XMP aminase (GMP synthetase) (Japanese Published Unexamined Patent Application No. 224498/85 corresponding to EP-A-0 185 092).

The process for producing GMP from XMP by means of an enzymatic reaction is based on the following reactions catalyzed by XMP aminase:

(1) XMP + ATP + $NH_3$

↓ XMP aminase

GMP + 5'-adenylic acid + pyrophosphoric acid

or

(2) XMP + ATP + L-glutamine

↓ XMP aminase

GMP + 5'-adenylic acid + L-glutamic acid.

Further, it has been reported that the activity of converting XMP to GMP is increased by endowing a microorganism belonging to the genus Brevibacterium with a resistance to decoyinine [Biotechnol. Bioengineer., 13, 229-240 (1971)].

However, in the conventional fermentation processes for the production of GMP, if the amount of microorganism is decreased according to the increase in XMP aminase activity, the ATP-reproducing activity becomes a rate-determining step. Therefore, in order to make good the shortage of ATP-reproducing activity and to enhance the GMP productivity, it is necessary either to add ATP to the medium, which is expensive, or to use a relatively large amount of ATP-producing microorganisms. This is true also of the process described in EP-A-0 185 092, where either ATP is added to the culture medium (Examples 1 to 3) or an E. coli transformant is used which itself has an ATP-reproduction ability and in which case ATP addition can be avoided (Examples 4 and 5).

In accordance with the present invention it has been discovered that the GMP yields of the E. coli transformants described in EP-A-0 185 092 can be substantially improved by coupling the reaction of the E. coli transformant with the reaction of an XMP/ATP producing microorganism of the genus Brevibacterium and Corynebacterium that coupled reaction taking place in a culture medium containing an organic solvent, a surfactant or a lytic enzyme.

Thus the present invention provides a process for the production of 5'-guanylic acid (GMP) from 5'-xanthylic acid (XMP) which comprises culturing an E. coli transformant carrying a recombinant DNA vector containing a DNA fragment containing a 5'-xanthylic acid aminase (5'-guanylic acid synthetase) gene and having the ability to produce GMP from XMP, ATP, ammonia and/or glutamine in a culture medium containing ammonia and/or glutamine, a source of carbon and other nutrients essential to the growth of the microorganism, including a source of phosphate ion and a source of magnesium ion, a source of XMP and ATP, and an organic solvent, surfactant or lytic enzyme, allowing the product GMP to accumulate in the culture medium, and recovering the accumulated GMP, wherein the source of XMP and ATP comprises an XMP and ATP-producing microorganism of the genus Brevibacterium or Corynebacterium having the ability to produce XMP in said medium for conversion into GMP by said transformant as well as the ability to convert AMP produced by the E. coli transformant back into ATP to act as the source of ATP for the said

transformant in the conversion of XMP into GMP and thereby coupling together the two reactions catalysed by the two microorganisms.

The XMP and ATP-producing microorganisms suitable for use in this invention include, for example, Brevibacterium ammoniagenes ATCC 21076, Brevibacterium ammoniagenes ATCC 21154, Brevibacterium ammoniagenes X-21 ATCC 21263, Corynebacterium glutamicum ATCC 15135, Corynebacterium glutamicum X-31 ATCC 21265, etc. By culturing these microorganisms according to an ordinary culturing procedure in an ordinary medium containing appropriate carbon sources, appropriate nitrogen sources, appropriate inorganic materials, amino acids, vitamins, etc., under aerobic conditions, while adjusting the temperature, pH, etc., a considerable amount of XMP can be accumulated in the medium as well as providing the source of ATP necessary for the conversion of XMP into GMP.

Suitable carbon sources for the XMP/ATP producing microorganisms include carbohydrates such as glucose, fructose, sucrose, maltose, etc.; sugar alcohols such as mannitol, sorbitol, glycerol, etc.; various organic acids such as pyruvic acid, lactic acid, citric acid, etc.; and various amino acids such as glutamic acid, methionine, lysine, etc. Other suitable sources include starch hydrolyzate, molasses, rice bran, cassava, bagasse and corn steep liquor.

Suitable nitrogen sources include ammonia; various inorganic and organic ammonium salts such as ammonium chloride, ammonium sulfate, ammonium carbonate, ammonium acetate, etc.; amino acids such as glutamic acid, glutamine, methionine, etc.; and nitrogen-containing organic materials such as peptone, NZ-amine, corn steep liquor, meat extract, yeast extract, casein hydrolyzate, and fish meal or its digested product.

Various other inorganic materials such as potassium dihydrogen phosphate, sodium monohydrogen phosphate, magnesium sulfate, sodium chloride, calcium chloride, iron chloride, copper sulfate, manganese chloride, ammonium molybdate, and zinc sulfate, can be added to the medium as may be necessary for the growth of the microorganism, as well as any necessary vitamins, amino acids, nucleic acids, etc. Naturally, if they are supplied by other medium components described above, it is not necessary to add those specific nutrients separately to the medium.

Culturing is generally carried out under aerobic conditions, for example, by shaking or by aeration and agitation. The preferred culturing temperature is generally 20 to 40 ° C, more preferably 25 to 35 ° C; and the pH of the medium is preferably maintained around neutrality during the culturing. Culturing period is usually 10 to 120 hours.

As the GMP-producing microorganism, any E. coli transformant can be used which carries a recombinant DNA fragment containing the 5'-xanthylic acid aminase (5'-guanylic acid synthetase) gene. One such transformant is Escherichia coli K294/pXA10, FERM BP-499, whose XMP aminase activity is increased by transformation with a plasmid containing the gene coding for the XMP aminase (guaA) of Escherichia coli K-12 inserted into the vector pBR322. The detailed procedures for construction of this plasmid, plasmid pXA10, and the subsequent transformation of the E. coli strain K294 are disclosed in EP-A-0 185 092, to which reference should be made.

These transformants can be cultured under substantially the same conditions and utilising the same carbon, nitrogen, inorganic mineral and other necessary nutrient sources as already described and listed above for the XMP/ATP-producing microorganism, save that for the conversion of XMP to GMP a specific source of ammonia and/or glutamine is required. In the case of E. coli K294/pXA10, FERM BP-499, especially good results are obtained if the tryptophan content of the medium is kept below 5 mg/l.

Culturing of the transformant is generally carried out under aerobic conditions, for example, by shaking or by aeration and agitation. The preferred culturing temperature is 20 to 50 ° C, more preferably 28 to 45 ° C. The pH is preferably maintained at about neutral. The culture period will usually be from 1 to 48 hours.

Various culturing techniques can be employed in the process of the present invention and involving either separate or simultaneous culturing of the two microorganisms. Thus, a fermentation liquor containing XMP, and a culture liquor of the E. coli transformant having an ability to convert XMP to GMP, can be mixed after completion of the individual culturing of the two microorganisms. Alternatively, a culture liquor containing the E. coli transformant can be added to the XMP fermentation medium at any time from the start to the completion of XMP fermentation, or vice versa. Yet again, the XMP-producing microorganism and the E. coli transformant can be cultured in a single medium at the same time.

To the culture broth containing XMP, XMP-fermenting cells and the transformed E. coli cells are added ammonia and/or glutamine, an ATP-reproducing substrate, and a surfactant, organic solvent, e.g. xylene, or lytic enzyme.

Conversion of XMP to GMP is carried out by adding phosphate ion and magnesium ion to the said mixture, while adjusting the pH to 6 to 10, preferably 7 to 8, and keeping the temperature at 20 to 50 ° C for

1 to 48 hours. The concentration of XMP during the conversion of XMP to GMP is desirably in a range of 1 to 100 mg/ml in terms of XMP•$Na_2$•$7H_2O$, which is hereinafter applied to express the concentration.

At this stage any suitable carbohydrates can be used, such as glucose, arabinose, lactose, maltose, sucrose, mannitol, sorbitol, trehalose, starch hydrolyzate, etc.; organic acids such as pyruvic acid, lactic acid, acetic acid, alpha-ketoglutaric acid, etc.; and amino acids such as glycine, alanine, aspartic acid and glutamic acid. Alternatively, phosphorylated compounds such as sodium or potassium salt of acetylphosphoric acid, carbamylphosphoric acid and creatinephosphoric acid can be used.

Suitable surfactants used include cationic surfactants such as polyoxyethylenestearylamine (for example, Nymeen S-215, product of Nihon Yushi), cetyltrimethylammonium bromide, etc.; anionic surfactants such as sodium oleylamide sulfate, etc.; and amphoteric surfactants such as polyoxyethylenesorbitan monostearate (for example, Nonion ST221, product of Nihon Yushi). Suitable surfactant concentrations range from 0.1 to 50 mg/ml, preferably 1 to 20 mg/ml.

Suitable organic solvents include toluene, xylene, aliphatic alcohols, benzene, and ethyl acetate, and can be used in concentrations of from 0.1 to 50 µl/ml, preferably 1 to 20 µl/ml.

During the conversion of XMP to GMP, the concentration of phosphate ion and magnesium ion is desirably kept in a range of 4 to 400 mM. Where necessary, these can be supplied or supplemented by the addition of suitable phosphate and/or magnesium salts to the culture medium. Suitable phosphate salts include the sodium, potassium and magnesium salts of phosphoric acid. As the magnesium salt, any salt of magnesium with an inorganic or organic acid can be used.

As the source of ammonia and/or glutamine, ammonia gas, inorganic and organic ammonium salts, glutamine, and glutamine-containing natural products such as yeast extract, casamino acid, corn steep liquor, etc. can be used.

Recovery of GMP from the culture broth can be carried out according to ordinary procedures using activated carbon, ion exchange resins, etc.

The process of the present invention is illustrated by the following examples.

Example 1

Step (a) One loopful of Escherichia coli K294/pXA10 (FERM BP-499) was inoculated in a sterilized 70 ml large test tube containing 10 ml of a seed medium (pH 7.2) comprising 1% polypeptone, 0.5% meat extract, 0.5% yeast extract and 0.25% sodium chloride, and cultured at 30°C for 16 hours by reciprocative shaker (280 reciprocation/min.). Then 2 ml of the cultured seed medium were inoculated in a 1 l Erlenmeyer flask containing 200 ml of M9 medium (pH 7.4) consisting of 6 mg/ml $Na_2HPO_4$, 3 mg/ml $KH_2PO_4$, 5 mg/ml NaCl, 1 mg/ml $NH_4Cl$, 4 µg/ml thiamine•HCl, 3 mg/ml glucose and 0.25 mg/ml $MgSO_4$•$7H_2O$, 2 mg/ml casamino acid, and cultured at 30°C for 16 hours by rotary shaker (220 rpm). The cells were collected by centrifugation at 13,000 x g for 20 minutes and frozen at -20°C for preservation.

Step (b) One loopful of Brevibacterium ammoniagenes ATCC 21154 was inoculated in a sterilized large test tube containing 10 ml of a seed medium consisting of 1% polypeptone, 0.5% meat extract, 0.5% yeast extract and 0.25% sodium chloride (pH 7.2) and cultured at 30°C for 24 hours by reciprocative shaking (280 reciprocation/min.). Then 2 ml of the cultured seed medium was inoculated in 300 ml Erlenmeyer flasks provided with baffles each containing 20 ml of a medium consisting of 15% glucose, 0.01% casein hydrolyzate, 0.7% yeast extract, 1.0% ammonium sulfate, 0.3% $KH_2PO_4$, 0.3% $K_2HPO_4$, 0.5% $MgSO_4$•$7H_2O$, 10 µg/ml each of adenine and guanine, and 10 µg/l of biotin (pH 7.2), autoclaved at 120°C for 20 minutes, and cultured at 30°C by rotary shaker (220 rpm), while the pH was kept approximately at pH 7 by adding urea to the medium, if necessary. At the 92nd hour from the start of culturing 38.0 mg/ml XMP was formed.

Step (c) The frozen Escherichia coli cells produced in step (a) were suspended in water and added to the XMP fermentation liquor produced in step (b) so that the wet cell concentration was 7.5 mg/ml. To that mixture were then added 50 mg/ml glucose, 2 mg/ml sodium phytate, 5 mg/ml $Na_2HPO_4$, 5 mg/ml MgSO4•$7H_2O$, 4 mg/ml Nymeen S-215 and 10 µl/ml xylene. 20 ml quantities of the thus obtained mixture were then poured into 200 ml beakers, and stirred by a magnetic stirrer at 900 rpm to conduct the conversion reaction of XMP to GMP at 42°C for 24 hours while adjusting the pH to about 7.4 by adding aqueous ammonia thereto. As a result, it was found that 32.0 mg/ml GMP (calculated as GMP•$Na_2$•$7H_2O$) was formed and accumulated in 23 hours.

Example 2

Example 1 was repeated, except that in place of the frozen Escherichia coli cells, 2 ml of the

Escherichia coli culture broth were added directly to 20 ml of Brevibacterium ammoniagenes culture broth containing 33 mg/ml XMP. The conversion of XMP to GMP in the combined culture broth was carried out in the same manner as in Example 1. As a result, 23.1 mg/ml GMP was formed.

Relevant deposit details of E. coli transformant referred to hereinbefore and described in EP-A-0 185 092 are as follows:

Escherichia coli K294/pXA10 deposited 8th March 1984 under the terms of the Budapest Treaty with the Fermentation Research Institute, Agency of Industrial Science and Technology, Japan, Deposit No. FERM BP-499.

## Claims

1. A process for the production of 5'-guanylic acid (GMP) from 5'-xanthylic acid (XMP) which comprises culturing an E. coli transformant carrying a recombinant DNA vector containing a DNA fragment containing a 5'-xanthylic acid aminase (5'-guanylic acid synthetase) gene and having the ability to produce GMP in the culture medium from XMP, ATP, ammonia and/or glutamine in a culture medium containing ammonia and/or glutamine, a source of carbon and other nutrients essential to the growth of the microorganism, including a source of phosphate ion and a source of magnesium ion, a source of XMP and ATP, and an organic solvent, surfactant or lytic enzyme, allowing the product GMP to accumulate in the culture medium, and recovering the accumulated GMP, characterised in that the source of XMP and ATP comprises an XMP and ATP-producing microorganism of the genus Brevibacterium or Corynebacterium having the ability to produce XMP in said medium for conversion into GMP by said transformant as well as the ability to convert AMP produced by the E. coli transformant back into ATP to act as the source of ATP for the said transformant in the conversion of XMP into GMP and thereby coupling together the two reactions catalysed by the two microorganisms.

2. A process according to claim 1, characterised in that the E. coli transformant is Escherichia coli K294/pXA10, FERM BP-499.

3. A process according to claim 1 or 2, characterised in that the XMP - and ATP-producing microorganism is
Brevibacterium ammoniagenes ATCC 21076
Brevibacterium ammoniagenes ATCC 21154
Brevibacterium ammoniagenes X-21 ATCC 21263
Corynebacterium glutamicum ATCC 15135
or Corynebacterium glutamicum X-31 ATCC 21265

4. A process according to any one of claims 1 to 3, characterised in that the culture medium contains as said surfactant a polyoxyethylenestearylamine and as an organic solvent xylene.

## Revendications

1. Procédé de production d'acide 5'-guanylique (GMP) à partir de l'acide 5'-xanthylique (XMP), qui comprend la culture d'un transformant d'E. coli portant un ADN vecteur recombinant, lequel comporte un fragment d'ADN contenant un gène codant pour l'acide 5'-xanthylique aminase (acide 5'-guanylique synthétase) et possède la capacité de produire du GMP dans le milieu de culture à partir d'XMP, d'ATP, d'ammoniac et/ou de glutamine, dans un milieu de culture contenant de l'ammoniac et/ou de la glutamine, une source de carbone et d'autres matières nutritives essentielles pour la croissance du microorganisme, y compris une source d'ions phosphates, une source d'ions magnésium et une source d'XMP et d'ATP, ainsi qu'un solvant organique, un tensio-actif ou une enzyme lytique, le fait de laisser le GMP produit s'accumuler dans le milieu de culture et la récupération du GMP accumulé, caractérisé en ce que la source d'XMP et d'ATP consiste en un microorganisme producteur d'XMP et d'ATP, appartenant au genre Brevibacterium ou Corynebacterium et possédant la capacité de produire, dans ledit milieu, de l'XMP qui sera converti en GMP par ledit transformant, ainsi que la capacité de reconvertir en ATP l'AMP produit par le transformant d'E. coli, de façon à jouer le rôle de source de l'ATP utilisé par ledit transformant pour la conversion d'XMP en GMP, ce qui fait que l'on opère un couplage entre les deux réactions catalysées par les deux microorganismes.

2. Procédé conforme à la revendication 1, caractérisé en ce que le transformant d'E. coli est Escherichia

coli K,294/pXA10, FERM BP-499.

3. Procédé conforme à la revendication 1 ou 2, caractérisé en ce que le microorganisme producteur d'XMP et d'ATP est
Brevibacterium ammoniagenes ATCC 21076,
Brevibacterium ammoniagenes ATCC 21154,
Brevibacterium ammoniagenes X-21 ATCC 21263,
Corynebacterium glutamicum ATCC 15135, ou
Corynebacterium glutamicum X-31 ATCC 21265.

4. Procédé conforme à une quelconque des revendications 1 à 3, caractérisé en ce que le milieu de culture contient une poly(oxyéthylène)-stéarylamine en tant que tensio-actif, et du xylène en tant que solvant organique.

## Patentansprüche

1. Verfahren zur Herstellung von 5'-Guanylsäure (GMP) aus 5'-Xanthylsäure (XMP) umfassend das Kultivieren einer E. coli-Transformante, die einen rekombinanten DNA-Vektor enthält, der ein 5'-Xanthylsäureaminase (5'-Guanylsäuresynthetase)-Gen enthaltendes DNA-Fragment enthält, und die die Fähigkeit zur Erzeugung von GMP aus XMP, ATP, Ammoniak und/oder Glutamin in einem Kulturmedium besitzt, in einem Ammoniak und/oder Glutamin, eine Kohlenstoffquelle sowie andere für das Wachstum der Mikroorganismen essentielle Nährstoffe einschließlich einer Phosphat- und Magnesiumionenquelle, einer XMP- und ATP-Quelle und ein organisches Lösungsmittel, grenzflächenaktives Mittel oder lytisches Enzym enthaltenden Kulturmedium, Anreichern von GMP im Kulturmedium und das Gewinnen des angereicherten GMP, **dadurch gekennzeichnet,** daß die XMP- und ATP-Quelle ein XMP und ATP bildender Mikroorganismus der Gattung Brevibacterium oder Corynebacterium ist, der die Fähigkeit zur Bildung von XMP für die Umwandlung in GMP durch die Transformante sowie die Fähigkeit zur Umwandlung von durch die E. coli-Transformante erzeugtem AMP zurück in ATP hat, um so als ATP-Quelle für die Transformante bei der Umwandlung vom XMP in GMP zu wirken und dabei die durch die zwei Mikroorganismen katalysierten beiden Reaktionen miteinander zu koppeln.

2. Verfahren nach Anspruch 1, dadurch gekennzeichnet, daß die E. coli-Transformante Escherichia coli K294/pXA10, FERM BP-499 ist.

3. Verfahren nach Anspruch 1 oder Anspruch 2, dadurch gekennzeichnet, daß der XMP und ATP bildende Mikroorganismus
Brevibacterium ammoniagenes ATCC 21076
Brevibacterium ammoniagenes ATCC 21154
Brevibacterium ammoniagenes X-21 ATCC 21263
Corynebacterium glutamicum ATCC 15135 oder
Corynebacterium glutamicum X-31 ATCC 21265
ist.

4. Verfahren nach einem der Ansprüche 1 bis 3, dadurch gekennzeichnet, daß das Kulturmedium ein Polyoxyethylenstearylamin als grenzflächenaktives Mittel und Xylol als organisches Lösungsmittel enthält.